# EUROPÄISCHE PATENTSCHRIFT

(11) **EP 0 962 217 B1**
(45) Veröffentlichungstag und Bekanntmachung des Hinweises auf die Patenterteilung: **30.11.2005**
(21) Anmeldenummer: 99109688.4
(22) Anmeldetag: 17.05.1999
(51) Int. Cl.: A61K 7/13

(54) **Verfahren zum oxidativen Färben von menschlichen Haaren**
Method for oxidative dying of human hair
Méthode pour teinture oxidative des cheveux humains

(30) Priorität: 05.06.1998 DE 19825134
(43) Veröffentlichungstag der Anmeldung: 08.12.1999
(73) Patentinhaber: KPSS-Kao Professional Salon Services GmbH, 64297 Darmstadt (DE)
(72) Erfinder: Lorenz, Heribert, 64401 Gross-Bieberau (DE)

(56) Entgegenhaltungen:
- EP-A- 0 462 883
- EP-B- 0 282 551
- WO-A-97/11673
- DE-A- 4 123 941
- DE-A- 4 219 981

## Beschreibung

Die vorliegende Erfindung betrifft ein neues Verfahren zum oxidativen Färben von menschlichen Kopfhaaren, das nicht nur haarschonender arbeitet als bisher bekannte und übliche Verfahren, sondern auch eine verbesserte Deckkraft und Farbintensität bzw. -stabilität bewirkt.

Die nach wie vor übliche Art der Haarfärbung ist die Färbung mit Oxidationsfarbstoffen, die unmittelbar vor der Anwendung mit Oxidationsmittel-Zusammensetzungen, insbesondere auf Basis von verdünntem Wasserstoffperoxid, vermischt und auf das Haar aufgetragen werden.

Der pH-Wert dieser gebrauchsfertigen Zusammensetzung liegt dabei in der Regel im alkalischen Bereich, insbesondere bei pH-Werten zwischen etwa 9 und etwa 11.

Durch diese alkalische Behandlung wird das Haar, insbesondere bei häufiger Wiederholung des Färbevorganges, in seiner Struktur geschädigt, vor allem, wenn es sich um bereits vorgeschädigtes Haar handelt. Dieses poröse Haar weist nach der Färbung auch eine schlechte Farbbeständigkeit auf.

Es wurde bereits vorgeschlagen, diese Nachteile der bisher üblichen Haarfärbung dadurch zu überwinden, daß, in Abkehr von der oben beschriebenen Praxis, anstelle der alkalisch eingestellten Zusammensetzungen aus Oxidationsfarbstoff und Oxidationsmittel eine analoge Zusammensetzung eingesetzt wird, deren pH-Wert im schwach sauren Bereich zwischen etwa 5,9 und 6,9 liegt.

Derartige Mittel, die z.B. in den DE-OSen Nr. 35 30 270, 36 28 397 und 36 28 398 beschrieben sind, haben sich in der Tat als wesentlich weniger haarschädigend als alkalische Produkte erwiesen.

In manchen Fällen, d.h., bei Einstellung spezieller Farbnuancen, läßt allerdings die Intensität und Stabilität der mit diesen verbesserten Zusammensetzungen erzielten Haarfärbungen etwas zu wünschen übrig.

Die Erfindung hat sich die Aufgabe gestellt, diese Nachteile zu überwinden.

Die Lösung dieser Aufgabe besteht in der Anwendung eines neuen Verfahrens nach Anspruch 1 zum oxidativen Färben menschlicher Haare.

Durch die Anwendung dieses Verfahrens wird eine schonende Färbung des Haares, jedoch gleichwohl auch eine ausgezeichnete Farbintensität erreicht.

EP-B-282551 offenbart ein Verfahren zur oxidativen Haarfärbung mittels alkalischer und saurer Zusammensetzungen, die auf getrennte Zonen der Haarfazer aufgetragen werden.

DE-A-4 123 941 offenbart ein Verfahren zur Haarfärbung mittels zweier alkalischer Zusammensetzungen.

WO-A-9 711 673 offenbart ein Verfahren zur Haarfärbung mittels saurer Zusammensetzungen.

Die Zeitspannen der Farbeinwirkung können durch die Applikation von Wärme, vorzugsweise 30 bis 50 °C, beispielsweise 40 °C, um etwa ein Viertel bis zur Hälfte reduziert werden.

Sowohl die in der ersten Behandlungsstufe zum Einsatz gelangenden, alkalischen eingestellten Oxidationsfarbstoffmischungen als auch die in der zweiten Behandlungsstufe verwendeten, sauer eingestellten Oxidationsfarbstoffmischungen, die jeweils mindestens je eine Entwickler- und je eine Kupplersubstanz sowie ein Oxidationsmittel enthalten, sind an sich bereits bekannt.

Bezüglich der alkalisch eingestellten gebrauchsfertigen (d.h., das Peroxid enthaltenden) Mischungen, deren pH-Wert zwischen 8 und 11, vorzugsweise 9 und 10, insbesondere bei 9,5 liegt, wird hierzu auf den Stand der Technik, z.B. auf die Monografie von K. Schrader, "Grundlagen und Rezepturen der Kosmetika", 2. Aufl., S. 784-804 (1989), verwiesen; die dort beschriebenen Produkte sind im Rahmen des erfindungsgemäßen Verfahrens ebenso einsetzbar wie die aus dem umfangreichen Stand der Technik bekannten weiteren Entwickler- und Kupplersubstanzen sowie Nuanceure.

Gleiches gilt hinsichtlich der sauer, d.h. auf einen pH-Wert von 2 bis 6,5, insbesondere 3 bis 5, eingestellten gebrauchsfertigen Farbstoffmischungen zur Durchrührung der zweiten Stufe des erfindungsgemäßen Verfahrens.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2-Hydroxyethylamino)-5-aminotoluol und 1-Amino-4-bis-(2'-hydroxyethyl)-aminobenzol bzw. deren wasserlösliche Salze; beispielhafte Kupplersubstanzen sind Resorcin, 2-Methylresorcin, 4-Chlorresorcin, 2-Amino-4-chlorphenol, 4-(N-methyl)aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 1,4-Diamino-2-chlor-benzol, 4,6-Dichlorresorcin, 1,3-Diaminotoluol, 1-Hydroxynaphthalin, 4-Hydroxy-1,2-methylendioxybenzol, 1,5-Dihydroxy-naphthalin, 1,7-Dihydroxynaphthalin, 2,7-Dihydroxynaphthalin, 4-Hydroxy-1,2-methylendioxybenzol, 2,4-Diamino-3-chlorphenol und/oder 1-Methoxy-2-amino-4-(2'-hydroxyethylamino)benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbzusammensetzungen kann jeweils etwa 0,25 bis etwa 5 Gew.-%, je nach gewünschter Färbung, betragen.

Als Oxidationsmittel werden vor allem verdünnte Wasserstoffperoxid-Lösungen, -Emulsionen oder -Gele eingesetzt, möglich, aber weniger üblich ist auch die Verwendung weiterer Peroxide wie Erdalkaliperoxiden, Harnstoffperoxid, Melaminperoxid, etc. in entsprechenden stöchiometrischen Mengen.

Die Oxidationsfarbstoffzusammensetzungen können als Lösungen, Cremes, Pasten, Gele, Aerosole, etc. Verwendung finden.

Vorzugsweise beträgt das Verhältnis der Menge von in der ersten Stufe aufgebrachtem alkalischen Färbemittel zu demin der zweiten Stufe aufgebrachten sauren Färbemittel etwa 1:3 bis 3:1, vorzugsweise etwa 1:1. Die Einwirkungszeit liegt bei jeweils fünf bis dreißig Minuten, insbesondere bei jeweils 10 bis 20, beispielsweise jeweils 15 Minuten. Das erfindungsgemäße Verfahren eignet sich dabei sowohl zum ganzheitlichen, d.h. erstmaligen Färben der Haare als auch zum Nachfärben.

Dabei kann es zweckmäßig sein, wenn die Einwirkung des alkalischen Färbemittels etwas länger dauert als diejenige des sauer eingestellten Mittels.

Der pH-Wert der auf dem Haar befindlichen Farbmischung nach Aufbringung des sauer eingestellten Haarfärbemittels liegt dabei vorzugsweise zwischen 5 und 8, insbesondere 6 bis 7,5.

In den folgenden Beispielen wird die Erfindung illustriert.

### Beispiel 1

Auf Strähnen gebleichten Menschenhaares wurde eine Färbezusammensetzung, die durch Vermischen von gleichen Gewichtsteilen einer 6%-igen Wasserstoffperoxidlösung und eines Oxidationsfarbstoffvorprodukts der folgenden Zusammensetzung erhalten wurde, und einen pH-Wert von 9,8 aufwies, aufgebracht.

| Trägermasse | |
|---|---|
| Cetylstearylalkohol | 11,00 (Gew.%) |
| Oleth-5 | 5,00 |
| Ölsäure | 2,50 |
| Stearinsäuremonoethanolamid | 2,50 |
| Cocosfettsäuremonoethanolamid | 2,50 |
| Natriumlaurylsulfat | 1,70 |
| Natriumsulfit | 1,00 |
| 1,2-Propandiol | 1,00 |
| Ascorbinsäure | 0,50 |
| Ammoniumchlorid | 0,50 |
| EDTA, Tetranatriumsalz | 0,20 |
| Parfum | 0,40 |
| Weizenproteinhydrolysat | 0,20 |
| Silica | 0,10 |

| Oxidationsfarbstoffmischung | |
|---|---|
| 2,5,6-Triamino-4-hydroxypyrimidinsulfat | 0,01 |
| 2,5-Diaminotoluolsulfat | 0,55 |
| 4-Chlorresorcin | 0,17 |
| Resorcin | 0,05 |
| 3-Aminophenol | 0,03 |
| Wasser | ad 100,00 |

Nach 15-minütiger Einwirkung wurde, ohne Auspülen, ein Farbprodukt aus gleichen Gewichtsteilen 6 %-iger H₂O₂-Lösung und einer analogen Oxidationsfarbstoffvorzusammensetzung, die jedoch mit Citronensäure auf einen pH-Wert von 5,0 eingestellt war, aufgebracht und nach weiterer 15-minütiger Einwirkung mit Wasser gut ausgespült und getrocknet.

Es wurde eine starke, intensive, ausdrucksvolle Mittelblondfärbung erhalten, die auch nach fünf Haarwäschen unverändert war.

Strähnen, die in gleicher Weise 30 Minuten nur mit der alkalischen Farbzusammensetzung behandelt worden waren, zeigten demgegenüber eine schwächere Färbung, die nach fünf Haarwäschen deutlich blasser war als die nach dem erfindungsgemäßen Verfahren erzielten. Darüberhinaus war auch der Griff des Haares nach der nur alkalischen Färbung deutlich rauher.

Eine 30-minütige Färbung lediglich mit der sauer eingestellten Farbzusammensetzung des erfindungsgemäßen Verfahrensschritts 2 führte zu einer wesentlich weniger intensiven und stabilen Haarfarbe.

### Beispiel 2

In eine der Zusammensetzung nach Beispiel 1 entsprechende Trägermasse wurde die folgende Oxidationsfarbstoffmischung eingebracht:

| | |
|---|---|
| 2,5,6-Triamino-4-hydroxypyrimidinsulfat | 0,01 (Gew.-%) |
| 2,5-Diaminotoluolsulfat | 0,90 |
| 2-Amino-4-hydroxypyridin | 0,80 |
| 1-Naphthol | 0,17 |
| 3-Aminophenol | 0,10 |
| Resorcin | 0,03 |

Dieses Produkt wurde mit 6 %-iger H₂O₂-Lösung im Gewichtsverhältnis 1:1 vermischt (pH 9,5) und auf Strähnen aus gebleichtem Menschenhaar aufgebracht.

Nach 15-minütiger Einwirkung wurde, ohne Ausspülen, eine mit Weinsäure auf einen pH-Wert von 6,0 eingestellte Mischung aus gleichen Gewichtsteilen des oben beschriebenen Oxidationsfarbstoffprodukts und 6%-iger H₂O₂-Lösung aufgebracht, nach weiterer 15-minütiger Einwirkung mit Wasser ausgewaschen, shampooniert und getrocknet.

Es wurde eine intensive, glänzende Mahagoni-Färbung erhalten, die auch nach fünf Haarwäschen noch stabil war.

Die gleiche Behandlung über 30 Minuten nur mit einer identischen sauren Farbzusammensetzung führte zu wesentlich schwächeren Ausfärbungen; nach Anwendung ausschließlich einer alkalischen Zusammensetzung wies das Haar nicht nur eine weniger intensive Färbung, sondern auch einen rauhen Griff auf.

### Beispiel 3

In eine der Zusammensetzung nach Beispiel 1 entsprechende Trägermasse wurde die folgende Oxidationsfarbstoffmischung eingebracht:

| | |
|---|---|
| 2,5-Diaminotoluolsulfat | 0,80 (Gew.-%) |
| Resorcin | 0,30 |
| 2-Amino-4-hydroxypyridin | 0,06 |
| 3-Aminophenol | 0,03 |

Dieses Produkt wurde mit 6%-iger H₂O₂-Lösung im Gewichtsverhältnis 1:1 gemischt (pH 9,6) und auf Strähnen aus gebleichtem Menschenhaar aufgebracht.

Nach 20-minütiger Einwirkung wurde, ohne Ausspülen, eine mit Citronensäure auf einen pH-Wert von 4,0 eingestellte Mischung aus gleichen Gewichtsteilen des oben beschriebenen Oxidationsfarbstoffprodukts und 6%-iger H₂O₂-Lösung aufgebracht, nach weiterer zehnminütiger Einwirkung gründlich ausgespült und getrocknet.

Es wurde eine intensive rotbraune Färbung erhalten, die auch nach fünf Haarwäschen noch stabil war, wobei das Haar einen weichen, lockeren Griff aufwies.

## Patentansprüche

1. Verfahren zum oxidativen Färben von menschlichen Haaren, **dadurch gekennzeichnet, daß** zunächst eine alkalisch eingestellte, mindestens eine Entwickler- und mindestens eine Kupplersubstanz sowie ein Oxidationsmittel enthaltende Oxidationsfarbstoffnischung die einen pH-wert von 8 bis 11 aufweist und anschließend, nach fünf- bis dreißigminütiger Einwirkung ohne zwischenzeitliches Spülen, eine sauer eingestellte einen pH-wert von 2 bis 6,5 aufweisende, mindestens eine Entwickler- und mindestens eine Kupplersubstanz sowie ein Oxidationsmittel enthaltende Oxidationsfarbmischung auf die Haare aufgebracht, und das Haar nach erfolgter Farbstoffeinwirkung gespült wird.

2. Verfahren nach Anspruch 1, **dadurch gekennzeichnet, daß** die Einwirkungsdauer des sauer eingestellte Mittels gleich lang oder kürzer ist als die Einwirkungsdauerdes alkalisch eingestellte Mittels.

3. Verfahren nach Anspruch 1 oder 2, **dadurch gekennzeichnet, daß** die Farbstoffeinwirkung bei 30 bis 50 °C erfolgt.

4. Verfahren nach Ansprüche 1 bis 3, **dadurch gekennzeichnet daß** die Farbmischung nach Aufbringen des sauer eingestellte Haarfärbemittels einen pH-wert von 5 bis 8 aufweist.

## Claims

1. Process for the oxidative dyeing of human hair, wherein first an alkaline oxidation dyestuff composition with a pH-value of 8 to 11, comprising at least one developing and at least one coupling substance as well as an oxidizing agent is applied to the hair, and subsequently, after five to thirty minutes processing, without rinsing, an acidic oxidation dyestuff composition with a pH-value of 2 to 6.5 comprising at least one developing and one coupling substance as well as an oxidizing agent is applied, and the hair is rinsed after processing of the dyestuff compositions.

2. Process according to claim 1, wherein the processing time of the acidic composition is equal to or shorter than the processing time of the alkaline composition.

3. Process according to claims 1 or 2, wherein the dyestuff composition is processed at 30° to about 50° C.

4. Process according to claims 1 to 3, **characterized in that** after applying the acidic composition the total dyeing composition has a pH-value of 5 to 8.

## Revendications

1. Procédé pour teindre par oxydation les cheveux humains, **caractérisé en ce qu'**on applique sur les cheveux d'abord un mélange de colorants par oxydation, rendu alcalin, présentant un pH de 8 à 11, contenant au moins une substance révélatrice et au moins une substance de couplage ainsi qu'un agent d'oxydation, et ensuite, après une action de cinq à trente minutes sans rinçage intermédiaire, un mélange de colorants par oxydation, rendu acide, présentant un pH de 2 à 6,5, contenant au moins une substance révélatrice et au moins une substance de couplage ainsi qu'un agent d'oxydation, et que l'on rince le cheveu après que l'action du colorant ait eu lieu.

2. Procédé selon la revendication 1, **caractérisé en ce que** la durée d'action du produit rendu acide est aussi longue ou plus courte que la durée d'action du produit rendu alcalin.

3. Procédé selon la revendication 1 ou 2, **caractérisé en ce que** l'action du colorant a lieu à une température de 30 à 50°C.

4. Procédé selon les revendications 1 à 3, **caractérisé en ce que** le mélange colorant présente une valeur de pH de 5 à 8 après l'application de la teinture capillaire rendue acide.
